# EUROPEAN PATENT APPLICATION

(11) **EP 1 355 155 A1**
(43) Date of publication of application: **22.10.2003**
(21) Application number: 03007820.8
(22) Date of filing: 04.04.2003
(51) Int. Cl.: G01N 33/543

(54) **Combinational biosensor**

(30) Priority: 04.04.2002 US 116348
(71) Applicant: TRW INC., Redondo Beach, CA 90278 (US)
(72) Inventor: Moats, Richard K., Annandale, VA 22003 (US); Sullivan, Brian M., Redondo Beach, CA 90278 (US)
(74) Representative: Schmidt, Steffen J., Dipl.-Ing.

(57) **Abstract**

The existence of any one of N² bioagents in a sample, N being an integer greater than 1, may be quickly determined in two major steps: (1) performing N separate ELISA processes (e.g. Test 1, Test 2, and Test 3, where N=3) on portions of the sample concurrently to identify (INDIC. A) a group of N bioagents that contain the bioagent, each of the N separate ELISA processes is capable of identifying the presence of a bioagent that falls within a unique group of N bioagents and in which the bioagents detectable by any one of those processes is different from the bioagents that are detectable by any other of the N processes. Then, when a test shows positive, performing N additional ELISA processes (e.g. Test 4, Test 5 & Test 6, where N=3) on portions of the sample concurrently to identify (INDIC. B) the particular bioagent. Each of the latter N processes is capable of only identifying a respective one of the individual bioagents that form the group of N bioagents identified in the previous step.

## Description

### REFERENCE TO PRIOR APPLICATIONS

Reference is made to U.S. Application, serial no 09/837,946, filed April 19, 2001, entitled "Automated Computer Controlled Reporter Device for Conducting Immunoassay and Molecular Biology Procedures" and to application, serial no. 10/055318, filed Oct. 23, 2001, entitled Combinational Strategy for Identification of Biological Agents, all of which are assigned to the assignee of the present application.

### FIELD OF THE INVENTION

This invention relates to detection of bioagents (and/or nucleic acids), and, more particularly, to a method for identifying the presence of bioagents and/or nucleic acids of specific interest in a specimen in the shortest possible time, with a minimum of necessary equipment and with known tests.

### BACKGROUND

Health authorities are ever alert to the outbreak of a contagious disease that, if not checked early, could grow to epidemic proportion amongst the general population. To that purpose, the health authorities are charged with monitoring the environment for the incidence of unusual illnesses, symptomatic of a disease, obtaining and analyzing samples to identify the biological agent causing the disease, and, once identified, publish protective measures that must be taken to halt the spread of the disease. Typically, biological agents, such as viruses and bacteria, are part of nature, and outbreaks of disease occur naturally. However, biological terrorism, the deliberate release of a harmful virus or bacteria or other biological agent (hereafter, "bioagent") amongst a general population, and bio-warfare, the deliberate release of a bioagent against military troops in battle, are also of concern.

One known test procedure or process for detection of a specific bioagent that is applicable to a variety of fields, such as biotechnology, environmental protection and public health, is the enzyme linked immunoassay (hereafter referred to as "ELISA"). The ELISA process constitutes an identification process that uses molecular interactions to uniquely identify target substances. A basic definition of ELISA is a quantitative in vitro test for an antibody or antigen (e.g., a bioagent) in which the test material is adsorbed on a surface and exposed to a complex of an enzyme linked to an antibody specific for the substance being tested for with a positive result indicated by a treatment yielding a color in proportion to the amount of antigen or antibody in the test material. The basic ELISA procedure is described more specifically, for one, in a book entitled Methods in Molecular Biology Vol 42, John R. Crowther, Humana Press, 1995.

The "antibody specific for the substance being tested for" in the foregoing definition constitutes a recognition molecule, a molecule that is restricted manner. That is, the recognition molecule binds to a specific three-dimensional structure of a molecule or to a two-dimensional surface that is electrically charged and/or hydrophobic in a specific surface pattern. It may also be recognized that ELISA-like approaches using other recognition molecules can also be used, such as aptamers, DNA, RNA and molecular imprint polymers.

More recently, the foregoing definition for ELISA has been expanded beyond the colormetric approach, wherein color and color intensity is used as a reporter or indicia of the antigen or antibody, to include a voltametric or amperiometric approach to detection, wherein a rate of change of voltage or current conductivity in proportion to the amount of antigen or antibody contained in the test material provides the indicia. Patent Cooperation Treaty application PCT/US98/16714, filed August 12, 1998 (International Publication No. WO 99/07870), entitled "Electrochemical Reporter System for Detecting Analytical Immunoassay and Molecular Biology Procedures" (hereafter the "16714 PCT application"), claiming priority of United States patent applications No. 09/105,538 and 09/105,539"), to which the reader may refer, describes both a colormetric and an electrochemical reporter system for detecting and quantifying enzymes and other bioagents in analytical and clinical applications. The electrochemical reporter system of the 16714 PCT application employs a sensor for detecting voltametric and/or amperiometric signals that are produced in proportion to the concentration of organic (or inorganic) reporter molecules by redox (e.g. reduction-oxidation) recycling at the sensor.

In brief, in the ELISA test, the suspect bioagent is initially placed in a water-based buffer, such as a phosphate buffered saline solution, to form a sample solution. That sample solution is mixed with a quantity of particles, such as beads, the surface of which is coated with an antibody to the suspect bioagent, herein also referred to as a recognition molecule and sometimes as a receptor molecule. The particular antibodies used to coat the beads are known to bind to the bioagent of interest or of concern and is a primary antibody or "1° Ab". That is, the antibody coating exhibits a chemical "stickiness" that is selective to specific bioagents.

Any bioagent present in the sample solution binds with a noncovalent bond to a respective antibody and thereby becomes attached to a respective one of the beads in the mixture-solution. If the sample solution does not contain a bioagent or if the bioagent that is present in the solution is not one that binds to the selected antibody, then nothing binds to the foregoing antibody. Further processing of the ELISA process then shows nothing.

Assuming the suspect bioagent is present in the sample, the bioagent bonds to the antibody that is coated on the beads. The solution then contains a quantity of bioagent molecules bound respectively to a like quantity of coated beads, a 1°Ab/bioagent combination. The mixture is optionally washed, as example, in a phosphate-buffered saline, and a second antibody, more specifically, an antibody and enzyme linked combination, is then added to the mixture. The second antibody is also one that is known to bind to the suspect bioagent, another recognition molecule. The second antibody may be either be one that is monoclonal, e.g. one that binds to only one specific molecule, or polyclonal, e.g. a mixture of different antibodies each of which shares the characteristic of bonding to the target bioagent. The enzyme, is covalently bound to the second antibody and forms a complex that is referred to as a secondary antibody-enzyme conjugate or "2°Ab-enz". As known by those skilled in the art, an enzyme is a "molecular scissors", a protein that catalyzes a biological reaction, a reaction that does not occur appreciably in the absence of the enzyme. The enzyme is selected to allow the subsequent production of an electrochemically active reporter.

The 2°Ab-enz binds to the exposed surface of the immobilized bioagent to form an "antibody sandwich" with the bioagent forming the middle layer of that sandwich. The antibody sandwich coated beads are washed again to wash away any excess 2°Ab-enz in the solution that remains unbound.

The beads and the attached antibody sandwich, the 1° Ab/bioagent/2°Ab-enz complex, in the solution are placed over the exposed surface of the redox recycling sensor. The substrate of the foregoing enzyme is added to the solution and the substrate is cleaved by the enzyme to produce an electrochemically active reporter. The substrate of the enzyme, referred to as PAP-GP, is any substance that reacts with an enzyme to modify the substrate. The effect of the enzyme is to separate, cut, the PAP, a para-amino phenol, the electrochemically active reporter, from the GP, an electrochemically inactive substance.

The foregoing chemical reaction is concentrated at the surface of the sensor. The rate of production of the foregoing reporter (PAP) is proportional to the initial concentration of bioagent. The reporter reacts at the surface of the sensor, producing an electrical current through the sensor that varies with time and is proportional to the concentration of the bioagent, referred to as redox recycling. The occurrence of the electric current constitutes a positive indication of the presence of the suspect bioagent in the sample. Analysis of the electric currents produced over an interval of time and comparison of the values of that electric current with existing laboratory standards of known bioagents allows quantification of the concentration of bioagent present in the initial sample.

The ELISA process has wide application in detecting bioagents. The greater the number of laboratories equipped to check for specific bioagents and the more widely those laboratories are dispersed over a geographic region, the more swiftly a bioagent occurring in the environment can be recognized and handled by the health authorities. Unfortunately, the number of such facilities is relatively small. Recognizing that dispersal of ELISA test capability among the general population, not just laboratories, is a desirable goal in bioagent detection, one finds a lack of trained personnel. which is also problematic. Wide dispersal of ELISA test capability may not as a practical matter be possible unless the ELISA test can be carried out by persons of lesser skill.

As example, the electrochemical ELISA procedure and apparatus of the cited 16714 PCT application and the predecessor ELISA procedures appear well suited to practice in a microbiology laboratory by highly skilled personnel who are alert to the details of the test process. Other facilities or environments in which such an analysis is desirable, however, may not enjoy either the availability of highly skilled technicians or an adequately equipped laboratory. In such environments, the availability of a foolproof, user-friendly test apparatus that is able to analyze a sample and report a meaningful result with minimal human intervention is certainly desirable.

Recognizing that need, one of the present inventors, together with other co-inventors, created an automated test procedure, which is described in U.S. patent application serial. No. 09/837,946, filed April 19, 2001, entitled Automated Computer Controlled Reporter Device for Conducting Immunoassay and Molecular Biology Procedures (hereafter the "946 application"), assigned to the assignee of the present application, the content of which is incorporated herein by reference. The apparatus of the '946 application provides a user friendly stand-alone portable unit that is able to automatically perform an ELISA test which may be operated by persons who are not biologists and who require minimal training. The automated system contains a number of solutions and pumps that are controlled by a programmed computer.

The foregoing system also employs beads of magnetic material that are coated with the recognition molecule and a magnetic positioning device to manipulate and position the coated magnetic beads under control of the computer, such as during the washing steps of the ELISA process, and in positioning the beads at the sensor during redox recycling. The automated test device of the '946 application provides a solution to dispersal of testing units that takes into account the lesser skills of prospective operators. However, like the prior manual procedure, the automated procedure did not determine which suspected virus should be sought or the priority to employ in the search to identify the suspect bioagent.

Each of the foregoing ELISA test procedures, whether manual or automatic, and/or colormetric or amperiometric, searches for a single suspect bioagent, and, if detected, determines the concentration of that bioagent. The identification is essentially a "go" or "no-go" procedure. In one approach to identification, the test procedure is repeated serially using different receptor molecules until the bioagent is identified. If the result of the one test is a "no go", then a second bioagent is made the suspect and the test is repeated for that second bioagent. The foregoing test procedure may be continued a great many times until either the particular bioagent is detected, one exhausts the supply of known receptor molecules or one exhausts the supply of known bioagents.

Even though the ELISA test is automated, as when employing the test apparatus of the cited '946 application, identification of a bioagent could take a great deal of time to accomplish if testing is accomplished in serial order, particularly if the bioagent turns out to be the least likely one in an extended list of suspect bioagents. One solution for reducing the time to identification is to utilize a bank of test apparatus, one test apparatus for each bioagent in the group of possibilities, and carry out the separate ELISA test procedures concurrently. Such an approach requires a great deal of equipment, particularly if one tests for a great number of different bioagents, and, basically, appears impractical. As example, if one is concerned about fifteen different bioagents as possibilities, it is possible to concurrently test using a bank of fifteen automated ELISA testers or other test apparatus, each catered to a respective bioagent. But so much test apparatus is expensive, requires a great deal of space in total or miniaturization, and would take much effort to stock with reagents and maintain. Hence, the approach is thought to be impractical.

One invention to solve the foregoing problem is presented in a prior U.S. application, serial no. 10/055318. filed Oct. 23, 2001, entitled Combinational Strategy for Identification of Biological Agents (hereafter the "'318 application"), naming one of the present inventors, the content of which is incorporated herein by reference. That invention explicitly recognizes that different recognition molecules (e.g. different types) may be grouped together and used concurrently in an ELISA test to determine if a bioagent is present that links to any one of those different recognition molecules, and, hence, falls within the group of corresponding bioagents. By the invention described in the '318 application, one of up to 2^{N}-1 bioagents in a sample is determined and identified by dividing the sample into N parts and performing N separate identification processes (e.g., ELISA), one process for each of the N parts, N being an integer greater than 1. Each of those N identification processes is assigned a respective group or combination of bioagents to identify. That group is unique to the respective identification process, with the bioagents of the group or combination being selected from the 2^{N}-1 bioagents and with the sum of those bioagents constituting that group or combination being 2^{(N-1)} bioagents, which is less than 2^{N}-1 bioagents.

Each such identification process is capable of identifying any one of a number of bioagents in the group or combination of bioagents assigned for detection to the respective identification process. At least some of the bioagents in the group or combination of bioagents assigned to one identification process are also shared by the group or combination of bioagents that are assigned for identification to at least one other one of the identification processes and each group or combination is assigned a bioagent that is unique to the respective identification process. Each of the N separate identification processes accordingly possess the capability of uniquely identifying a respective single one of the bioagents from the 2^{N}-1 bioagents combination that none of the other identification processes is capable of identifying. By use of combinational logic a particular bioagent is readily identified.

As example, an embodiment in which N equals two, the number of bioagents that can be detected using two ELISA processes is three (i.e. 2²-1). Thus the sample containing one of the bioagents (or none) is parsed in two (i.e. N) parts and each of those parts is separately tested for the bioagents (i.e. N tests). The one ELISA process to test a parsed sample is prepared so that the process is capable of identifying only bioagents A and B; the other ELISA process is prepared to be capable of identifying only bioagents B and C, whereby the identification of bioagent B, common to both processes, is shared. Further, only one of those two processes is uniquely capable of identifying bioagent A, and the other process is uniquely capable of identifying bioagent C. Thus, if both identification processes identify a bioagent, one interpretation is that the bioagent in the sample is B; otherwise, only one of the two identification processes will identify bioagent A or C if present. Another interpretation is that a combination of bioagents is present, such as both A and C, which is possible, and is an ambiguity. Then another test, such as for either A or C, is taken to resolve any such ambiguity. Essentially in a single major step using two ELISA test apparatus simultaneously, two possible bioagents may be identified at one time and a third may be determined free of any ambiguity with a follow-on test, reducing the necessity for use of three test apparatus or reducing the time for a single test apparatus to perform three separate tests. As the number of bioagents increases, so grows the savings. As further example, given thirty-one bioagents of interest, only five test apparatus operating concurrently are needed to identify the particular bioagent in a sample, reducing the need for twenty-six test apparatus, concurrently operating, or reducing the time required from that required to perform thirty-one tests with a single test apparatus.

The present invention possesses essentially the same general advantages as the invention of the '318 application and accomplishes a like purpose, but in a different way. As the number of bioagents one wishes to detect and identify increases, the potential amount of ambiguity resulting from positive test results obtained from the test apparatus in the invention of the '318 application increases greatly. Reducing the complexity of the testing procedure and making that procedure faster and more inexpensive to set up, operate and maintain without significantly reducing the advantages obtained by the invention of the '318 application is obviously desirable. As an advantage the present invention accomplishes that purpose.

Accordingly, an object of the present invention is to reduce the number of individual tests that must be performed to identify the presence of one of multiple suspect bioagents in a sample, and to reduce the time required to make such identification without the complexity of the prior combinational testing procedure.

A further object of the invention is to provide a procedure for identifying a bioagent in a sample that is among the bioagents that are included in a predefined list of bioagents.

A further object of the invention is to provide the means to search for specific bioagents with increased speed and efficiency.

And, a still further object of the invention is define a unique user-friendly protocol for quickly identifying an unknown bioagent in a sample using the ELISA process.

### SUMMARY OF THE INVENTION

In accordance with the foregoing, the method quickly determines the existence of any one of N² bioagents in a sample, in two major steps, N being an integer greater than 1: (1) First, performing N separate ELISA processes on respective portions of the sample concurrently to identify a group of N bioagents that contain the bioagent, each of the N separate ELISA processes being capable of identifying the presence of a bioagent that falls within a unique group of N bioagents and in which the bioagents detectable by any one of those separate N processes is different from the bioagents that are detectable by any other of the N processes; and then (2), when a test shows positive, performing N additional ELISA processes on portions of the sample concurrently to identify the particular bioagent; each of the latter N processes being capable of only identifying a respective one of the individual bioagents that form the group of N bioagents identified positive in the previous step.

The foregoing and additional objects and advantages of the invention, together with the structure characteristic thereof, which were only briefly summarized in the foregoing passages, will become more apparent to those skilled in the art upon reading the detailed description of a preferred embodiment of the invention, which follows in this specification, taken together with the illustrations thereof presented in the accompanying drawings.

### BRIEF DESCRIPTION OF THE DRAWINGS

Figures 1 and 2 are high level pictorial illustrations of an embodiment for identifying a bioagent from amongst nine different possible bioagents, respectively representing the primary and secondary procedures used to identify the bioagent.

Figure 3 illustrates the embodiment of Figs. 1 and 2 in greater detail.

### DETAILED DESCRIPTION OF THE PREFERRED EMBODIMENTS

The following description assumes a familiarity with the ELISA process described in the preceding background and, in greater detail in the '16714 PCT application and the '946 application. A starting point in an ELISA test is to coat receptor molecules on beads. Specifically the beads are coated with a receptor molecule (e.g., recognition molecule or, as variously termed "1°Ab") that adsorbs a specific antigen (hereafter referred to as a bioagent). Likewise, the ELISA test requires a secondary antibody-enzyme conjugate, specific to the bioagent (e.g., 2°Ab-enz). The ELISA test for that specific bioagent determines both the presence of that bioagent in the solution, and, if present, the concentration of the bioagent. The purpose of the present invention is to identify the presence of a suspect bioagent from a plurality of predetermined candidates.

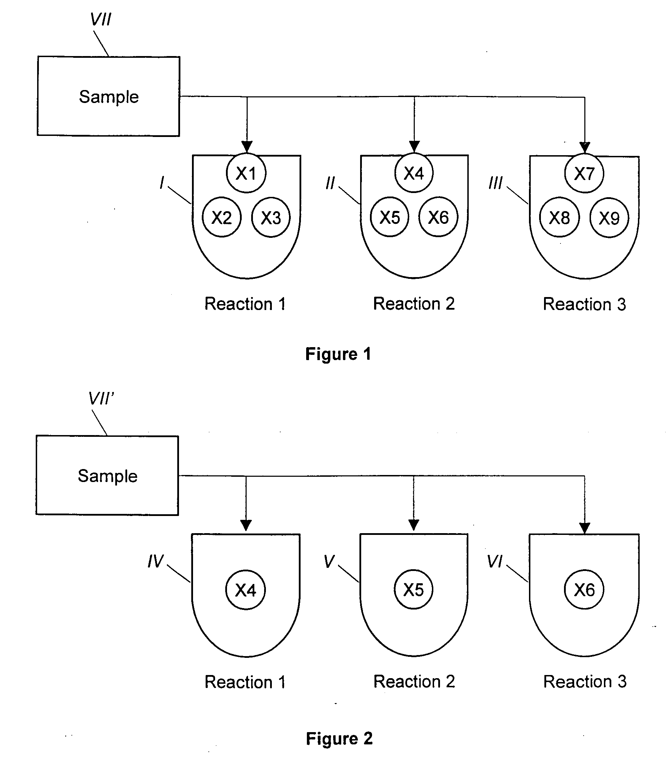

Figures 1 and 2 are high level pictorial illustrations of an embodiment for identifying a bioagent from amongst nine different possible bioagents, respectively representing the primary and secondary procedures used to identify the bioagent. For convenience, the nomenclature used to denominate the elements in those figures is considered. In this description, the letter X1 represents a bead coating (e.g. the recognition molecule) that serves as a receptor for a specific bioagent (and may also be used to represent the secondary antibody-enzyme conjugate specific to the bioagent (e.g., 2°Ab-enz). The specific bioagent is given a corresponding designation AX1. As example, the bead coatings for three bioagents AX1, AX2 and AX3 are represented as X1, X2 and X3.

To run an ELISA test for three bioagents using a conventional ELISA test procedure, it is necessary to have three separate recognition molecules for those bioagents, accomplished, for example, with a first vial with beads coated with receptor molecule X1, another vial of beads coated with receptor molecule X2 and a third vial coated with receptor molecule X3. It is also necessary in the ELISA process to have the three separate secondary antibody-enzyme conjugate (e.g., 2°Ab-enz) specific to the bioagent associated with AX1, AX2 and AX3. A given sample of the bioagent is divided into three parts and placed in the corresponding vial of coated beads. Each vial is tested in turn using the ELISA procedure to determine the existence of the corresponding agent in the sample and, if present, the concentration of each. The present invention and method employs that prior technique as a secondary identification procedure, illustrated in Fig. 2.

Alternatively, if one is using the ELISA test to merely determine if any one of bioagents AX1, AX2 and AX3 is present in a sample without identifying the specific bioagent, one may combine coated beads from each of the foregoing three vials in a single vial to form a single coated bead mixture or bead set. Then the ELISA test is run on that mixture using the appropriate antibody-enzyme linked combinations for each of the three agents simultaneously. The foregoing may be referred to as a "combined" test procedure. The foregoing does not identify which bioagent is present, only that at least one of the three bioagents is present. The foregoing combined procedure is described in the '318 application and is used in the invention presented in that application. As becomes apparent from the description, that combined testing, illustrated in Fig. 1, is incorporated in the present invention and method.

In a primary procedure of the new method, a sample containing a bioagent is tested (e.g. the primary tests or procedure) for the presence of several groups of possible bioagents, as later herein-described in greater detail. If a group tests positive, then additional individual tests (the secondary procedures) are made of the sample to identify the individual bioagent within the group.

The high level pictorial illustrations showing the primary and secondary steps or procedures used to identify an individual bioagent contained in a sample, presented in Figs. 1 and 2, respectively, illustrate a specific embodiment that contains three separate ELISA procedures in a primary procedure, shown in Fig. 1, and a secondary procedure, shown in Fig. 2 that together permit identification of as many as nine different bioagents. Referring to Fig. 1, three vials I. II and III contain the recognition molecules for the nine different bioagents that were selected by the operator in setting up the equipment. Those recognition molecules are sorted among the three vials so that the three vials contain equal numbers (e.g. types) of recognition molecules. Vial I contains the recognition molecules X1, X2 and X3 for bioagents Ax1, Ax2 and Ax3; vial II contains the recognition molecules X4, X5 and X6 for bioagents Ax4, Ax5 and Ax6 and vial III contains the recognition molecules X7, X8 and X9 for bioagents Ax7, Ax8 and Ax9. Effectively the individual recognition molecules for the nine different pre-selected bioagents of concern are formed into distinct groups, each of which is unique. Each such group in turn is formed of individual recognition molecules that are also unique; that is, the recognition molecule in a particular vial, such as X4 is not included in any other vial.

The operator maintains a record correlating the respective vials and the bioagents represented by those vials. Sample VII containing the suspect bioagents, typically a water based buffer solution with the suspect bioagent, is portioned and one of those portions is divided into three parts. Each of those three sample parts is deposited in a respective one of the three vials to potentially produce a reaction (e.g. linkage) with a recognition molecule at the commencement of ELISA processing.

Fig. 2 represents the second part of the test. Assuming one of the nine bioagents in the collection is present in one of the three vials, and, specifically, for purposes of this description, is within vial II, then the contents of vial II tests positive by the respective ELISA process. Since the recognition molecules, X4, X5 and X6, included in that vial (and, hence, the corresponding bioagents) are known, the sample is then tested for each of those individual bioagents. Recognition molecule X4 is deposited in vial IV, recognition molecule X5 is deposited in vial V and recognition molecule X6 is deposited in the third vial VI.

Another portion VII' of the sample containing the bioagent is divided into three parts and each part is placed within a respective one of the three vials to potentially produce a linking reaction with a recognition molecule. Since each vial contains a recognition molecule unique to a particular bioagent, a reaction occurs in only the vial that contains the recognition molecule for the bioagent included in the sample. Each of the vials is subjected to an ELISA process, one particular vial tests positive, as example vial V, and the particular bioagent, here assumed to be bioagent AX5, is identified.

The advantage of evenly dividing the recognition molecules into groups that are equal in size is evident. If the recognition molecules for the nine possible bioagents in the foregoing embodiment were divided in the ratio: 4: 3: 2 to form the three groups, and if the suspect agent were located within the group that contains the four different recognition molecules, then the secondary testing requires four separate ELISA testing apparatus (or four ELISA tests) instead of the three tests that were described and illustrated for the 3:3:3 division. That is less efficient and undesirable.

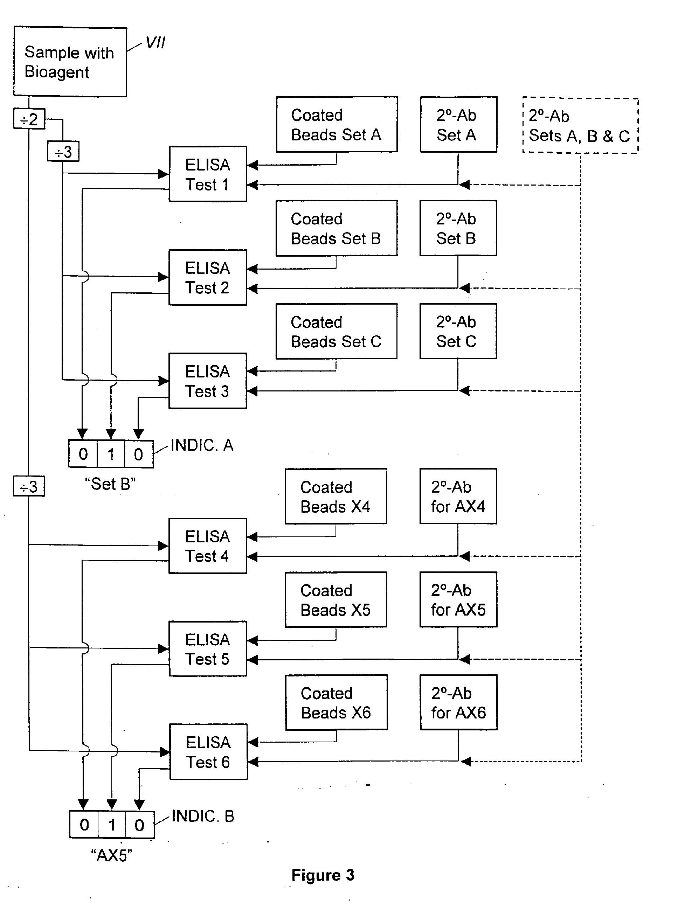

The foregoing is illustrated in more detail in Fig. 3 to which reference is made. Tests 1, 2 and 3 correspond with the procedure of Fig. 1 and Tests 4, 5 and 6 correspond to that of Fig. 2. Sample VII is initially divided into at least two portions, one of which is used in the primary procedure and the remainder reserved for use later, as needed. The one portion is divided amongst each of the ELISA tests and each portion of those three portions is deposited in respective vials or other containers holding the respective groups of recognition molecules. ELISA test 1 uses recognition molecules, collectively identified as set A, which are X1, X2 and X3 in Fig. 1, and the appropriate conjugate, identified as 2°-Ab set A. Other known elements customary to an ELISA process, such as the PAP-GP reporter molecules and other details are not illustrated and need not be further described. ELISA test 2 includes the recognition molecules, collectively identified as set B, and the appropriate conjugate, identified as 2°-Ab set B and ELISA test 3 includes the recognition molecules, collectively identified as set C, and the appropriate conjugate, identified as 2°-Ab set C.

Each bead set is a mixture of differently coated beads. As example, to formulate a single mixture of beads that may be tested for the presence of more than one type of bioagent simultaneously such as three agents in one group, namely agents AX1, AX2 and AX3, multiple batches of coated beads are prepared, one batch of which is coated with receptor molecules X1, a second batch coated with receptor molecules X2 and a third batch coated with receptor molecules X3. Then, as example, equal portions of each batch are mixed together to form a bead mixture or, as otherwise termed, bead set. The conjugates for each bioagent group is also prepared as a mixture of the appropriate individual conjugates for the respective bioagents.

As an alternative to using separate coated beads, a bead may be coated with a number of different recognition molecules to form a multi-coated bead, as example, with recognition molecules X1, X2 and X3, producing an equivalent to the foregoing bead mixture. That alternative, which falls within the scope of the present invention, is the subject of another less preferred embodiment later herein described.

The foregoing bead mixture (or in the embodiment of a multi-coated bead, the bead) may be represented by a letter and descriptors defining the contents of the mixture, as example, A (X1, X2, X3) or represented in the form of a chart of the bead set, which follows, and the agents AX1, AX2 and AX3 that are to be detected by the test of that bead set. For additional clarity of this description, the preface AX is omitted from the numerical designation assigned to the bioagent in the included charts, identifying those bioagents simply as 1, 2 and 3. The descriptors X1, X2, X3 to the bead set, are also omitted, identifying the bead mixture or set simply by letter, A, since the receptor coatings may be inferred from the identification of the bioagents listed in the following chart.

In the described embodiment, bead mixture A is coated with recognition molecules that are receptive to bioagents 1, 2, and 3 (e.g., A(X1, X2, X3)), bead mixture B is coated with recognition molecules that are receptive to bioagents 4, 5 and 6 (e.g., B(X4, X5, X6)) and bead mixture C is coated with recognition molecules that are receptive to bioagents 7, 8 and 9 (e.g. C(X7, X8, X9)). The bead set and bioagents of this specific embodiment are represented in one chart as Case I.

### CASE I - Three Bead Sets & Three Primary/Secondary Tests

| Bead set | Bioagents to be detected by the bead set | | |
|---|---|---|---|
| A | 1 | 2 | 3 |
| B | 4 | 5 | 6 |
| C | 7 | 8 | 9 |

In the interest of obtaining a result quickly, tests for sets A, B and C are performed concurrently and the result of each test indicated, such as on an indicator, INDIC.A, illustrated in Fig. 3. The indication illustrated of 010 indicates that the bioagent falls within the group corresponding to the recognition molecules in set B, and that sets A and C are not present.

Once it is determined that a particular vial tests positive for a bioagent, one knows that the bioagent included in the sample is one of the group of bioagents represented by the specific recognition molecules originally placed in that vial. The next step is to test the sample individually for each of the bioagents in that group, which is the secondary procedure earlier referred to in connection with Fig. 2. Tests 4, 5 and 6 in Fig. 3 represent those tests. Another portion of sample VII containing the bioagent is divided into three parts, and each part is inputted into a respective ELISA test. Since a positive indication occurred using set B in the primary procedure, representing the recognition molecules for bioagents AX4, AX5 and AX6, the secondary testing is individually for those bioagents.

The operator has on hand a supply of nine groups of coated beads each group of which is coated with the recognition molecules for a respective one of the nine candidate bioagents and also maintains a like supply of the individual conjugates for each bioagent. From that supply ELISA test 4 is equipped with recognition molecule coated beads X4 for the 1°Ab in the testing and the conjugate for that bioagent, 2° Ab for AX4, is used in that test; ELISA test 5 is equipped with recognition molecule coated beads X5 for the 1°Ab in the testing and the conjugate for that bioagent, 2° Ab for AX5, is used in that test and ELISA test 6 is equipped with recognition molecule coated beads X6 for the 1°Ab in the testing and the conjugate for that bioagent, 2° Ab for AX6, is used in that test. The tests are preferably run concurrently to reduce delay in securing a result. The result is reported, such as on an indicator INDIC.B, which shows a "010" indicating identification of the AX5 bioagent.

It should be understood that the ELISA processes referred to in this description may be accomplished manually or automatically, and may be either of a type in which the outcome is a change in color of the solution, a change in electrical current, a change in electro-chemical luminescence or any other reporter system known or hereafter created that may be applied to the ELISA process as an improvement. Another variant is to use recognition molecules that are able to covalently attach to their target, such as photo-aptamers. Another improvement to the ELISA system proposes to use a non-specific capture agent instead of the recognition molecule coated beads. The recognition molecule that serves as that non-specific capture agent is general purpose in character and is capable of linking to a large number of different bioagents. Because that agent is non-specific, the enzyme linked recognition molecule must be specific to the particular bioagent or bioagents being addressed in the test. Conversely, another approach being developed is to employ a non-specific labeling strategy. That is a reporter molecule that is capable of linking to a large number of different bioagents. For that system, the recognition molecule of the coated beads should be specific to the bioagent or bioagents of interest. At present, the testing is preferably accomplished by the automated test system of the '946 application, being currently available, in which case the beads are formed of magnetic material and the outcome is a measurable change in electric current.

Apart from the use of recognition molecules in identification testing, it should be realized that the invention is independent of the specific manner or type of testing used. As example, the foregoing automated ELISA system employs the phenomenon of redox recycling at an electrode for generation of measurable electrical current (e.g. reporting). A more recent measurement technique employs electro-chemi-luminescence ("ECL") instead of redox recycling and may be substituted for the redox recycling function in the automated system without any material effect to the present invention. The ECL technique employs measurement of electro-chemical luminescence that is emitted as the reporter. That emission is measurable and serves as an indicator of the bioagent and the concentration.

In the preceding description, it may be implied that the recognition molecule employed in the conjugate for a specific bioagent used in the second part of the test was always identical to the recognition molecule for that bioagent used in the first part of the identification process. That is not necessarily the case. One finds that there are a number of different recognition molecules that recognize a single bioagent. Thus, in the second part of the identification process recognition molecules may be used for the bioagents in the group that differ in structure from the recognition molecules for those bioagents used in the first part of the identification process. Such an alternative is included within the scope of the present invention.

Moreover, some recognition molecules for a particular bioagent, as is known, discriminate between strains of a particular bioagent. As example, Anthrax is a bioagent that is available in different strains, such as the recently publicized Ames strain. Those different strains are characterized by different physical differences in the geometry or "code" of the molecule, which is a three-dimensional figure on a microscopic scale. Thus, one recognition molecule may link to a specific surface geometry of the bioagent that is available on one strain of the bioagent, but that surface geometry is not found on a different strain and the recognition molecule cannot link to that strain. The foregoing property is useful with the present invention. As example, if the primary procedure shows the possible presence of a bioagent, such as anthrax, that, is anthrax falls within the group of bioagents, and if it is known that one strain of anthrax is not regarded as serious, then one may employ a particular recognition molecule for anthrax during the secondary procedure that ignores the non-serious strain. Should the second part of the test result in all vials testing negative, then the operator knows that anthrax was present, but was the non-serious kind. The test identified the bioagent in a negative way.

Additionally, the foregoing embodiment was capable of testing for as many as nine different bioagents. If a fourth ELISA testing apparatus is added so that four tests may be conducted concurrently, it is possible to test for as many as sixteen different bioagents. Each of the four tests of the primary procedure would test for four different groups of four different bioagents, as indicated in the chart labeled Case II. Four tests are used in the primary procedure and four tests are used in each secondary procedure, locating the bioagent in two major steps. Likewise if two additional ELISA testers are added for a total of five, then testing may be accomplished for as many as twenty five different bioagents as indicated in the chart labeled Case III. Five tests are used in the primary procedure and five tests are used in each secondary procedure, again identifying the bioagent in just two major steps. Case IV identifies four possible bioagents.

### CASE II - Four Bead Sets & Four Primary/Secondary Tests

| Bead set | Bioagents to be detected by the bead set | | | |
|---|---|---|---|---|
| A | 1 | 2 | 3 | 4 |
| B | 5 | 6 | 7 | 8 |
| C | 9 | 10 | 11 | 12 |
| D | 13 | 14 | 15 | 16 |

### CASE III - Five Bead Sets & Five Primary/Secondary Tests

| Bead set | Bioagents to be detected by the bead set | | | | |
|---|---|---|---|---|---|
| A | 1 | 2 | 3 | 4 | 5 |
| B | 6 | 7 | 8 | 9 | 10 |
| C | 11 | 12 | 13 | 14 | 15 |
| D | 16 | 17 | 18 | 19 | 20 |
| E | 21 | 22 | 23 | 24 | 25 |

### CASE IV - Two Beads & Two Primary/Secondary Tests

| Bead set | Bioagents to be detected by the bead set | |
|---|---|---|
| A | 1 | 2 |
| B | 3 | 4 |

As one appreciates, the number of bioagents that can be detected depends on the number of ELISA testers available. Generalizing, given N such ELISA testers, as many as N² bioagents can be identified through performance of the two principal steps, the primary one to identify a group of bioagents that includes the yet unidentified bioagent and secondary ones to identify the particular bioagent within the group or groups that showed a positive response in the first test.

The number of agents detected can be increased even beyond the twenty-five given in the foregoing examples to greater numbers, depending on the willingness to provide additional testing apparatus for concurrent testing. Each such increase in the number of test apparatus increases N by one. There is a practical limit to such expansion, however. That limit depends on the sensitivity of the measurement equipment, the sensitivity of the measurement equipment to detect color when colormetric testing is used in the ELISA process, or the sensitivity of the measurement equipment to detect current and/or voltage during redox recycling when amperometric detection is used in the ELISA process.

As one appreciates, testing for multiple bioagents in a single reaction can decrease the sensitivity for the individual bioagents. At present the limit to sensitivity of bead-based systems is believed to be twenty-five to one hundred bioagents or approximately five to ten agents per reaction. However, as the technology of measurement apparatus improves or alternatives to beads are developed in the future that number may be increased further.

It should also be understood from the foregoing discussion that the number of different bioagents that may be detected, N², in the primary and secondary tests is a maximum number, as example, nine in Case I, sixteen in Case II, and twenty five in Case III and so on, are maximum numbers of bioagents. Therefore, it is always possible to actually test for a lesser number of different bioagents, although the particular testing arrangement being used is capable of testing for a greater number of different bioagents.

As example, the apparatus used for Case I is capable of identifying nine different bioagents. If, however, the operator is only interested in eight bioagents (more than the four possible for the apparatus of Case IV), one need only prepare and use the receptor molecules (and the corresponding enzyme linked receptor molecules) for those eight bioagents and consider the remaining one a dummy or placebo. The method would be followed, employing the three ELISA tests during the primary step. Instead of evenly distributing the different recognition molecules during testing in the primary step, such as 3:3:3 as in the case of nine bioagents, which is not possible since there are only eight different recognition molecules needed, the eight different recognition molecules would be distributed 3:3:2 amongst the three testing apparatus. In the secondary procedure only two individual ELISA tests would be required if the positive indication occurs in the vial containing the recognition molecules that correspond to the two bioagents.

In the foregoing embodiment, a mixture of beads was used to provide the recognition molecules for a number of different bioagents. It is also possible to coat a single bead with recognition molecules for many different bioagents and to run the ELISA process as in the prior embodiment. As example, considering the initial case given of determining the existence of any of nine possible bioagents in a sample in three ELISA processes, the primary test for the presence of any of bioagents AX1, AX2 and AX3 (alternately identified in the chart as bioagents 1, 2 and 3) in one of the three groups could be run on a single vial of beads, A, in which all the beads in the vial are all coated with three receptor molecules X1, X2 and X3 for bioagents 1, 2 and 3.

Consider further Case I, earlier discussed, in which any one of nine bioagents AX1 through AX9, arranged in three groups, are to be detected and identified using three ELISA tests that are run in parallel for which bead mixtures A, B and C were used. Bead mixture A contained beads coated, respectively, with recognition molecules for bioagents AX1, AX2 and AX3; and that mixture was formed by mixing beads from three separate vials, one of which contained the recognition molecules for bioagent AX1, the second of which contained the recognition molecules of bioagent AX2, and the third, the recognition molecules for bioagent AX3. By coating all the beads with all three recognition molecules to form bead set A, only a single vial of coated beads needs to be maintained. The same holds true for the beads that form bead mixtures B and C. The three recognition molecules for bioagents AX4, AX5 and AX6 may be coated on all the beads that define bead set B, and only a single vial of beads needs to be maintained. Bead set C is similarly formed with the three recognition molecules for bioagents AX7, AX8 and AX9.

The ELISA test operates on the foregoing multi-coated beads in the same manner, and obtains the same result as earlier described for the mixture of beads, which need not be repeated. As is clear from an understanding of the description of the invention, the foregoing alternative is equivalent to the procedure earlier described and falls within the scope of the present invention. It should thus be recognized that the term "bead mixture" and "beads" should be interpreted as being synonymous in the claims that follow, unless the context requires separate meaning.

Along similar lines the recognition molecules to link to the group of bioagents in the first phase of the test and to link to the individual bioagents in the second phase of the test may be replaced by a non-specific capture agent, earlier briefly mentioned, if such capture agent is available for the bioagents of interest. The recognition molecule that serves as that non-specific capture agent is general purpose in character and is capable of linking to a large number of different bioagents, as example, to any or all of the bioagents in bead sets A, B and C. The conjugate sets, 2°-Ab set A, 2°-Ab set B and 2°-Ab set C used in the first phase of the test, would each link to any of the specific bioagents to which the respective conjugate is designed to link. Likewise in the second part of the test the conjugates are specific to individual bioagents. As is apparent, the use of a general purpose capture agent reduces stock keeping requirements for the test apparatus.

It may also be desirable to reduce the number of conjugate solutions required for conducting the separate ELISA processes of the portions of the sample to minimize maintenance requirements and stock keeping of supplies. Referring again to Fig. 3 and Case I (e.g., N equals 3) earlier described, for this additional embodiment the conjugate solutions for the coated beads in the ELISA tests of the primary procedure, shown in the boxes labeled 2°-Ab set A, 2°-Ab set B and 2°-Ab set C are replaced by the conjugate solutions for the receptor molecules used on the three sets of coated beads A, B and C, shown in the dash line box labeled 2°-Ab sets A, B & C. Further, in the secondary ELISA tests, tests 4, 5 and 6, to identify a specific bioagent, the conjugate solutions for coated beads X4, X5 and X6, shown in the box labeled 2°-Ab for AX4, 2°-Ab for AX5 and 2°-Ab for AX6 are also replaced by the conjugate solutions in the dash line box labeled 2°-Ab sets A, B & C containing a conjugate solution for any of the recognition molecules.

In the running of ELISA test 1 on the sample, the individual receptor (2°Ab-enz) molecules of the conjugate solutions are able to bind only to the same bioagent molecules to which the coated beads in bead set A are able to bind. In Case 1, bead set A is able to bind only to bioagents 1 and 2, whereas the receptor molecules in the new conjugate solution are able to bind to bioagents 1, 2 and 3. However, since there is no coated bead to which the conjugate solution component for bioagent 3 is able to bind, nothing can happen, and that component of the conjugate solution is essentially neutral. The remaining components of the solution, the components for bioagents 1 and 2 react just as the components reacted in Case I. The additional component (or, in the case of Cases II and III, components) of the conjugate solution are essentially surplus. The same effect occurs in the running of ELISA test 2. In the latter test the component of the conjugate solution that is unique to bioagent 1 is surplus, since the beads are coated with receptor molecules only for bioagents 2 and 3 and, the remaining components of the conjugate solution that are unique to bioagents 2 and 3 react just as those components reacted in Case I earlier described.

As an alternative to the universal conjugate bead set described, an approach earlier briefly referred to is to employ a non-specific labeling agent for the conjugate. That is, the enzyme-linked recognition molecule used in the conjugate is capable of linking to a large number of different bioagents. For that system, the recognition molecule of the coated beads is specific to the bioagent or bioagents of interest

As one appreciates, in the foregoing embodiment and in the alternative last mentioned one is able to compound a single conjugate solution for use in all of the ELISA tests performed on a sample. The savings of time and stock keeping becomes more significant in the additional cases in which four, five or more ELISA tests are run concurrently, in which circumstance the conjugate solution can contain as many as sixteen, twenty-five or more components, respectively. Adopting the technique is particularly attractive when employing the Automated ELISA apparatus of the earlier cited '946 application, which makes the apparatus described therein easier for unskilled persons to use and maintain.

As one appreciates, the foregoing method and apparatus is easily expandable. Should the number of bioagents of concern increase over the years beyond the maximum possible with an existing apparatus, all one need do at most is add another testing apparatus for concurrent testing in the primary step, and add the additional recognition molecules and conjugates for the additional bioagents to the ELISA test apparatus. The complicated permutations and combinations required to do so with the apparatus of the '318 application are not necessary.

In addition to identifying one bioagent from among N² candidates, the advantages of simplicity and ease of use characteristic of the invention, is also useful when more than one bioagent is present. Such a situation is even thought to be likely to occur in some warfare scenarios. Reference is again made to Figs. 1 and 2 and the embodiment of the invention in which nine different bioagents are targeted for detection and identification. Assume that the sample contains bioagents AX3 and AX4. The recognition molecules for those bioagents X3 and X4 reside in separate vials, I and II, hence, as a result of the primary procedure both vials I and II produce a positive result. The operator would rightly conclude that at least two bioagents are present. Then a secondary procedure is performed on the sample (i.e. additional portions of the sample) using the recognition molecules X1, X2, and X3 associated with the first vial. The individual secondary ELISA tests identify bioagent AX3 as the culprit. Then a second secondary procedure is run on the sample (i.e. additional portions of the sample) using the recognition molecules X4, X5 and X6 contained in vial II. The individual secondary ELISA tests identify bioagent AX4 as the culprit. By extending to one more series of N ELISA tests both bioagents are identified. No changes in the apparatus is required and no further training of personnel is required.

Even less extra work is required if by chance only one vial tests positive during the primary procedure, as example if the sample contains bioagents AX2 and AX3. The operator might not immediately recognize that two bioagents are present. However, when the secondary procedures are performed using the recognition molecules X1, X2 and X3 in individual tests, both AX2 and AX3 will be readily identified. The logic of the foregoing testing can be extended further to three bioagents and being present in the sample, with the worst consequence being the repetition of the secondary procedures.

As used herein, when reference was made herein to "numbers" of recognition molecules or different recognition molecules it should be understood that the applicant's are referring to types or kinds of recognition molecules, and not to the quantity or volume of recognition molecules.

The ELISA process is recognized as an identification process that is or can be applied in many different fields, not only to identify bioagents, but nucleic acids as well. However, lacking terminology that is generic to and/or encompasses both bioagents and nucleic acids, it should be understood that the reference to bioagents used herein and in the claims that follow in this application includes nucleic acids. Further, identification processes equivalent to ELISA may be developed that use other recognition molecules, such as aptamers, DNA, RNA and molecular imprint polymers, and, hence, are ELISA-like in nature. The method described herein may be employed for those procedures. Thus for the purposes of this invention, the term ELISA should be construed to encompass all such ELISA-like procedures.

It is believed that the foregoing description of the preferred embodiments of the invention is sufficient in detail to enable one skilled in the art to make and use the invention without undue experimentation. However, it is expressly understood that the detail of the elements comprising the embodiment presented for the foregoing purpose is not intended to limit the scope of the invention in any way, in as much as equivalents to those elements and other modifications thereof, all of which come within the scope of the invention, will become apparent to those skilled in the art upon reading this specification. Thus, the invention is to be broadly construed within the full scope of the appended claims.

## Claims

1. The method of determining the existence of and identifying any one of up to a plurality of N² bioagents in a sample containing a bioagent, where N is an integer greater than 1, comprising the steps of:
(A) performing a separate detection process on each of N parts of the sample to define a plurality of N detection processes, said detection process employing molecular interactions to uniquely identify bioagents, said plurality of N detection processes possessing the capability collectively of identifying any of N² bioagents,
wherein each of said separate detection processes of said plurality of N detection processes possesses a capability to detect any of N different bioagents from a collection of up to N² known bioagents, and
wherein the bioagents that are detectable by any one of said separate detection processes in said plurality of N detection processes differs from any of the bioagents that are detectable by any other of said separate detection processes in said plurality of N detection processes, whereby a positive result of one of said N detection processes identifies a group of N bioagents that includes the bioagent to be identified; and
(B) performing a separate detection process on each of an additional N parts of the sample to define a second plurality of N detection processes, said detection process employing molecular interactions to uniquely identify bioagents,
wherein said second plurality of N detection processes possessing the capability collectively of identifying any of the N bioagents in said group of N bioagents identified by said positive result, and
wherein each of said separate detection processes of said second plurality of N detection processes possesses a capability to detect only a respective one of the bioagents from within said group of N bioagents identified by said positive result, whereby the bioagent in said sample is identified.

2. The method of determining the existence of and identifying any one of up to a plurality of N² bioagents in a sample containing a bioagent as defined in claim 1, further comprising the step, prior to steps (A) and (B), of:
(C) dividing a portion of the sample into at least 2N parts.

3. The method of determining the existence of and identifying any one of up to a plurality of N² bioagents in a sample containing a bioagent as defined in claim 1, further comprising the steps of:
(C) dividing a portion of the sample into N parts prior to step (A); and
(D) dividing another portion of said sample into an additional N parts prior to step (B).

4. The method of determining the existence of and identifying any one of up to a plurality of N² bioagents in a sample containing a bioagent, where N is an integer greater than 1, comprising the steps of:
(A) dividing a portion of the sample into N parts;
(B) performing a separate detection process on each of the N parts of the sample to define a plurality of N detection processes, said detection process employing molecular interactions to uniquely identify bioagents, said plurality of N detection processes possessing the capability collectively of identifying any of N² bioagents,
wherein each of said separate detection processes of said plurality of N detection processes possesses a capability to detect any of N different bioagents from a collection of up to N² known bioagents, and
wherein the bioagents that are detectable by any one of said separate detection processes in said plurality of N detection processes differs from any of the bioagents that are detectable by any other of said separate detection processes in said plurality of N detection processes, whereby a positive result of one of said N detection processes identifies a group of N bioagents that includes the bioagent to be identified;
(C) dividing another portion of the sample into N parts; and
(D) performing a separate detection process on each of the N parts of the second portion of said sample to define a second plurality of N detection processes, said detection process employing molecular interactions to uniquely identify bioagents,
said second plurality of N detection processes possessing the capability collectively of identifying any of the N bioagents in said group of N bioagents identified by said positive result, and
wherein each of said separate detection processes of said second plurality of N detection processes possesses a capability to detect only a respective one of the bioagents from within said group of N bioagents identified by said positive result, whereby the bioagent in said sample is identified.

5. The method of determining the existence of and identifying any one of a plurality of N² bioagents in a sample containing a bioagent as defined in claim 4, wherein said step of performing a separate detection process on each of said N parts to define a plurality of N detection processes comprises the step of performing said plurality of N detection processes concurrently.

6. The method of determining the existence of and identifying any one of a plurality of N² bioagents in a sample containing a bioagent as defined in claim 5, wherein each of said plurality of N detection processes comprises an enzyme linked immunoassay ("ELISA") process.

7. The method of determining the existence of and identifying any one of a plurality of N² bioagents in a sample containing a bioagent as defined in claim 6, wherein N is 2.

8. The method of determining the existence of and identifying any one of a plurality of N² bioagents in a sample containing a bioagent as defined in claim 6, wherein N is 3.

9. The method of determining the existence of and identifying any one of a plurality of N² bioagents in a sample containing a bioagent as defined in claim 6, wherein N is 4.

10. The method of determining the existence of and identifying any one of a plurality of N² bioagents in a sample containing a bioagent as defined in claim 6, wherein N is 5.

11. The method of determining the existence of and identifying any one of a plurality of N² bioagents in a sample containing a bioagent as defined in claim 4, wherein said step of performing said separate identification process on each of said N parts includes the steps of:
coating beads in N different collections and coating each collection of beads in said N different collections with recognition molecules for less than N² multiple bioagents but in which said N different collections contain collectively receptor molecules for all of said N² multiple bioagents, with one of said receptor molecules in each collection being a receptor for the same bioagent, and with another of said receptor molecules in each collection being unique amongst the receptor molecules of all other collections; and
applying each collection of coated beads in a respective one of said separate identification processes.

12. The method of determining the existence of and identifying any one of a plurality of N² bioagents in a sample containing a bioagent, where N is a number selected from the series of integers comprising 2, 3, 4, 5....x, comprising the steps of:
dividing a portion of the sample into N parts; and
performing a separate enzyme linked immunoassay ("ELISA") process on each of the N parts concurrently to detect if said bioagent is one that is within a predetermined group of bioagents that the respective ELISA process is able to detect,
each of said N separate ELISA processes possessing the capability of detecting a respective unique group of bioagents, and each detectable bioagent in each said group being unique;
dividing another portion of the sample into an additional N parts; and
performing a separate ELISA process on each of the additional N parts to detect an individual bioagent in that predetermined group of bioagents;
said step of performing a separate enzyme linked immunoassay ("ELISA") process on each of said N parts, including the steps of:
coating beads in N different groups and coating each group of beads in said N different groups with receptor molecules for N unique bioagents; and
said step of performing a separate enzyme linked immunoassay ("ELISA") process on each of said additional N parts, including the steps of:
coating individual beads with receptor molecules for N unique bioagents.
